# EUROPÄISCHE PATENTSCHRIFT

(11) **EP 2 779 899 B1**
(45) Veröffentlichungstag und Bekanntmachung des Hinweises auf die Patenterteilung: **28.10.2020**
(21) Anmeldenummer: 12798137.1
(22) Anmeldetag: 17.10.2012
(51) Int. Cl.: A61B 5/107

(54) **VORRICHTUNG ZUR LÄNGENMESSUNG**
HEIGHT MEASURING DEVICE
DISPOSITIF DE MESURE DE LONGUEUR

(30) Priorität: 15.11.2011 DE 102011118810
(43) Veröffentlichungstag der Anmeldung: 24.09.2014
(73) Patentinhaber: seca ag, 4153 Reinach BL 1 (CH)
(72) Erfinder: VON MAYDELL, Marc-Oliver, 22337 Hamburg (DE)
(74) Vertreter: Klickow & Wetzel PartGmbB
(86) Internationale Anmeldenummer: PCT/DE2012/001025
(87) Internationale Veröffentlichungsnummer: WO 2013/071904

(56) Entgegenhaltungen:
- WO-A1-2011/127578
- DE-U1-202010 016 423
- US-A1- 2005 171 451
- US-A1- 2010 298 708
- US-A1- 2010 312 143

## Beschreibung

Die Erfindung betrifft eine Vorrichtung zur Messung der Körperlänge einer Person, bei der im Bereich einer Innenbegrenzung eines Gebäuderaumes mindestens ein Emitter zur Abgabe eines Primärsignales und mindestens ein Sensor zur Erfassung eines Messsignals angeordnet sind und bei der sowohl der Emitter als auch der Sensor mit einer Steuereinrichtung gekoppelt sind, die mit einer Anzeigeeinrichtung gekoppelt ist.

Zur Messung der Körperlänge einer Person werden häufig sogenannte Längenmessstäbe eingesetzt, die beispielsweise selbststehend mit einem Fußteil versehen sind oder an einer Wand montiert werden. Die zu vermessende Person stellt sich typischerweise mit dem Rücken gegen diesen Längenmessstab und eine exakte Kopfpositionierung wird durch einen sogenannten Kopfanschlag vorgegeben. Die zu vermessende Person kommt hierdurch in einen Körperkontakt mit der Messeinrichtung, was häufig als unangenehm oder zumindest unerwünscht empfunden wird.

Bekannt sind ebenfalls bereits berührungslose Längen- oder Abstandsmesseinrichtungen, die im Bereich des Fußbodens eines Raumes aufgestellt werden. Hierdurch können Entfernungen, beispielsweise über eine Ultraschall-Messung, ermittelt werden.

Aus der US 2010/312143 A1 ist bereits eine Vorrichtung zur Messung der Körperlänge einer Person bekannt. Die Messung erfolgt unter Verwendung eines in einem Gebäuderaum angeordneten Emitters sowie eines Sensors. Vergleichbare Vorrichtungen zur Messung der Körperlänge einer Person werden auch in der WO 2011/127578 A1, der US 2005/171451 A1, der US 2010/298708 A1 und der DE 20 2010 016 423 U1 beschrieben.

Aus der WO 2011/127578 A1 ist bereits eine Vorrichtung zur Messung der Körperlänge einer Person bekannt Es werden ein Emitter und ein Sensor verwendet. Der Emitter ist als ein Schallgeber ausgebildet und der Sensor ist zum Empfang eines reflektierten Schalls geeignet.

Aufgabe der vorliegenden Erfindung ist es, eine Vorrichtung der einleitend genannten Art derart zu konstruieren, dass eine berührungslose Messung der Körperlänge bei einfacher Bedienbarkeit unterstützt wird.

Diese Aufgabe wird erfindungsgemäß durch die Merkmale von Patentanspruch Nr. 1 gelöst.

Der Begriff des Sensors ist im Zusammenhang mit der vorliegenden Erfindung umfassend zu verstehen. Insbesondere ist daran gedacht, dass der Sensor aus einem oder mehreren Sendern sowie aus einem oder mehreren Empfängern bestehen kann. Ebenfalls ist es möglich, die Funktion des Senders und des Empfängers im Bereich des Sensors durch eine einzige Komponente zu realisieren.

Durch die Anordnung sowohl des Emitters als auch des Sensors im Bereich der Innenbegrenzung eines Gebäuderaumes wird eine stationäre Einrichtung bereitgestellt, die eine sehr bedienungsfreundliche Benutzung unterstützt. Insbesondere sind sowohl der Emitter als auch der Sensor außerhalb von Bereichen angeordnet, die typischerweise Verschmutzungen unterliegen. Die Vorrichtung zur berührungslosen Messung der Körperlänge eignet sich somit insbesondere auch für Verwendungen im Bereich von Arztpraxen oder von Krankenhäusern, wo besonders hohe Anforderungen an die Sauberkeit gestellt werden.

Zusätzliche Informationen über die zu vermessende Person können dadurch bereitgestellt werden, dass die Vorrichtung zusätzlich eine Waage zur Erfassung eines Gewichtes der zu vermessenden Person aufweist.

Zur Anpassung an räumliche Gegebenheiten wird vorgeschlagen, dass die Steuereinrichtung zur Auswertung einer Referenzmessung ausgebildet ist, die den Abstand des Sensors zu einer Standfläche der zu vermessenden Person berücksichtigt.

Gemäß einer typischen Ausführungsform ist vorgesehen, dass der Sensor im Bereich einer Decke des Raumes angeordnet ist.

Gemäß einer anderen Ausführungsform ist es auch möglich, dass der Sensor im Bereich einer Wand des Raumes angeordnet ist.

Eine individuelle Anpassung an konkret vorliegende räumliche Gegebenheiten wird dadurch unterstützt, dass der Sensor unter Verwendung einer Halterung installiert ist.

Zur Unterstützung möglichst genauer Messergebnisse wird vorgeschlagen, dass ein Projektor zur Projektion einer Standmarkierung für die zu vermessende Person verwendet ist.

In den Zeichnungen sind Ausführungsbeispiele der Erfindung schematisch dargestellt. Es zeigen:
- Fig. 1: Eine schematische Darstellung einer Seitenansicht einer im Bereich einer Gebäudedecke angeordneten Messeinrichtung sowie einer zu vermessenden Person,
- Fig. 2: die Anordnung gemäß Fig. 2 bei zusätzlicher Verwendung eines Gestells für die Messeinrichtung,
- Fig. 3: eine schematische Darstellung einer weiteren Ausführungsform, bei der die Messeinrichtung im Bereich einer Wand des Gebäudes montiert ist,
- Fig. 4: ein weiteres Beispiel mit einer in einem Bereich einer Gebäudewand angeordneten Messeinrichtung mit seitlicher Messung,
- Fig. 5: eine Abwandlung der Messeinrichtung gemäß Fig. 1 bei Verwendung einer zusätzlichen Projektionseinrichtung zur Generierung einer Markierung im Bereich eines Fußbodens des Gebäudes und
- Fig. 6: ein schematisches Blockschaltbild zur Veranschaulichung der Kopplung des Emitters und des Sensors mit der Steuereinrichtung sowie der Verwendung einer Anzeigeeinrichtung.

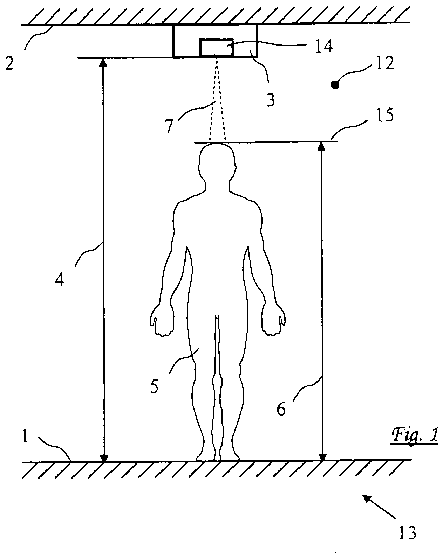

Gemäß der in Fig. 1 veranschaulichten Ausführungsform wird ein Innenraum (12) eines Gebäudes (13) zumindest bereichsweise von einem Fußboden (1) und einer Decke (2) begrenzt. Im Bereich der Decke (2) sind bei dem dargestellten Ausführungsbeispiel ein Emitter (14) sowie mindestens ein Sensor (3) angeordnet. Bei der Durchführung von Längen- oder Abstandsmessungen unter Verwendung des Emitters (14) sowie des Sensors (3) ist ein Abstand (4) zwischen dem Sensor (3) und dem Fußböden (1) zu berücksichtigen. Eine obere Begrenzung (15) eines Kopfes der zu vermessenden Person (5) weist zum Fußboden (1) einen Abstand auf, der der zu bestimmenden Körperlänge (6) entspricht.

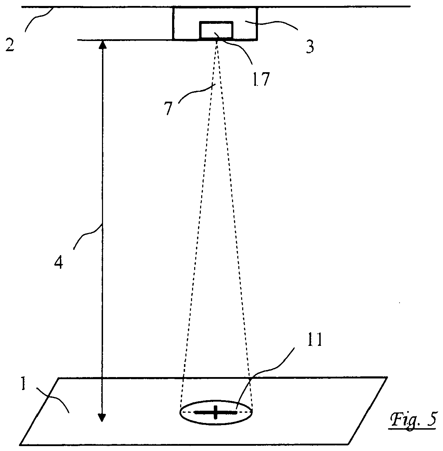

Fig. 1 und Fig. 5 veranschaulichen beispielhaft auch einen typischen Erfassungsbereich (7) des Sensors (3).

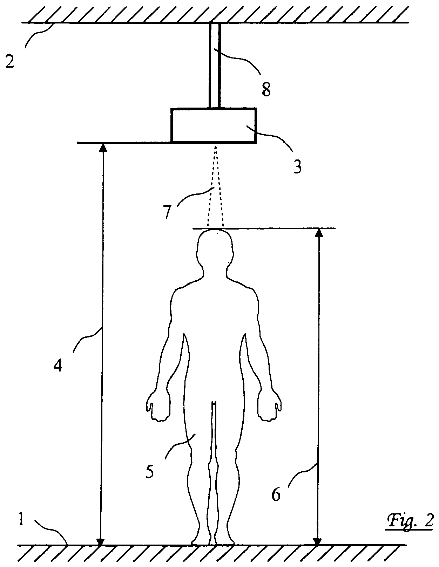

Fig. 2 zeigt eine gegenüber Fig. 1 abgewandelte Ausführungsform bei zusätzlicher Verwendung eines Distanzstückes (8) als Halterung für den Sensor (3). Das DistanzstOck (8) kann beispielsweise bei Raumhöhen verwendet werden, die zu einem zu großen Abstand zwischen dem Sensor (3) und der zu vermessenden Person (5) führen würden. Bei abgewandelten Ausführungsformen kann das Distanzstück (8) aber auch allgemein als Halterung für den Sensor (3) und/oder den Emitter (14) ausgebildet sein.

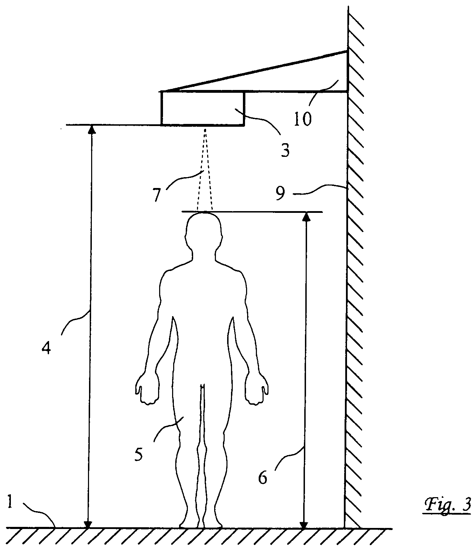

Fig. 3 zeigt eine weitere Ausführungsform, bei der der Sensor (3) im Bereich einer Wand (9) des Gebäudes (13) befestigt ist. Das dargestellte Ausführungsbieispiel zeigt die Befestigung des Sensors (3) unter Verwendung eines Wandhalters (10). Gemäß der Ausführungsform in Fig. 3 erfolgt ähnlich wie mit den Ausführungsformen in Fig. 1 und 2 eine Messung der Körperlänge bei einer Erfassungsrichtung in lotrechter Richtung nach unten. Der Wandhalter (10) dient somit im Wesentlichen zur geeigneten Positionierung des Sensors (3).

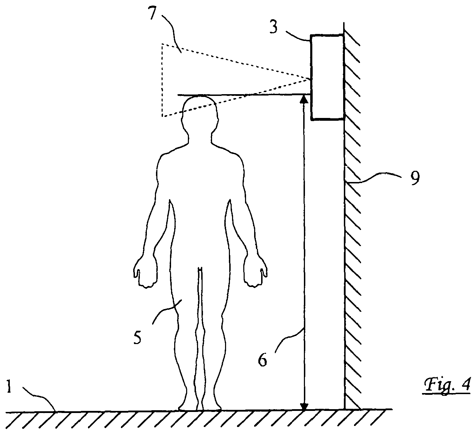

Gemäß des Beispiels in Fig. 4 ist der Erfassungsbereich (7) ausgehend vom Sensor (3) mit einer horizontalen Komponente ausgerichtet. Es erfolgt somit eine seitliche Messung der Körperlänge der zu vermessenden Person (5).

Gemäß der Erfindung in Fig. 5 wird ein Projektor (17) verwendet, der eine Markierung (11) im Bereich des Fußbodens (1) projiziert. Die Markierung legt einen optimalen Standpunkt für die zu vermessende Person (5) fest.

Ein Projektor für die Markierung kann beispielsweise in der Nähe des Sensors (3) angeordnet sein. Darüber hinaus ist es aber auch nicht erfindungsgemäß möglich, dass eine Projektion der Markierung von der Seite her erfolgen kann. Eine Projektion von der Seite vermeidet eine Abschattung der Markierung durch die zu vermessende Person bei einem Betreten der Markierung. Bei einer Anordnung des Projektors im Bereich des Sensors (3) kann der Projektor in das Gehäuse des Sensors (3) integriert werden.

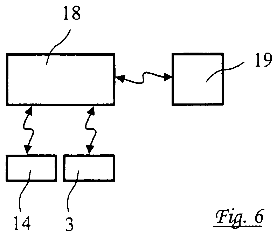

Fig. 6 veranschaulicht schematisch eine Kopplung des Emitters (14) sowie des Sensors (3) mit einer Steuereinrichtung (18). Die Kopplung erfolgt vorzugsweise berührungslos beispielsweise über eine Funkstrecke oder eine optische Kopplung. Die Steuereinrichtung (18) ist darüber hinaus mit einer Anzeigeeinrichtung (19) gekoppelt. Auch hier erfolgt die Kopplung vorzugsweise berührungslos.

Als Anzeigeeinrichtung (19) kann beispielsweise das Display eines Computers verwendet werden. Gedacht ist auch an die Verwendung von tragbaren in der Hand zu haltenden Einrichtungen, beispielsweise entsprechend einem Handy oder einer Fernbedienung.

In Abhängigkeit vom jeweiligen Anwendungsfall kann der Emitter (14) unterschiedliche Primärsignale abgeben, beispielsweise Ultraschall, Radarwellen oder einen Laserstrahl. Ebenfalls ist es möglich, den Emitter (14) lediglich als Beleuchtungseinrichtung auszubilden und als Sensor (3) eine Kamera zu verwenden, die vorzugsweise mit einer Steuereinrichtung gekoppelt ist, die zur Durchführung von Mustererkennungen ausgebildet ist.

Alternativ zur Verwendung eines speziellen Projektors (17) zur Generierung einer Markierung (11) im Bereich des Fußbodens (1), beispielsweise unter Verwendung eines roten Laserstrahles, ist es auch möglich, die Markierung (11) beispielsweise auf den Fußboden (1) aufzukleben oder hier in anderer Art und Weise dauerhaft zu erzeugen.

Durch die bereits erwähnte messtechnische Erfassung des Abstandes (4) als zu berücksichtigende Raumhöhe kann die Messeinrichtung in unterschiedlich hohen Räumen verwendet werden und die Steuereinrichtung (18) kann die derart ermittelte Längenreferenz als Korrekturwert berücksichtigen. Ebenfalls ist es möglich, beispielsweise eine Drift des Sensors (3) durch Temperaturänderungen oder durch Änderungen der Luftfeuchtigkeit durch eine oder mehrere Referenzmessungen zu berücksichtigen. Auch diese Korrekturwerte können bei der exakten Ermittlung der jeweiligen Körperlänge der zu vermessenden Person (5) berücksichtigt werden.

Bei seitlichen Längenmessungen kann beispielsweise eine Triangulation zum Einsatz kommen.

Eine Energieversorgung der verwendeten Gerätekomponenten kann über das vorhandene Stromnetz oder Ober Batterie beziehungsweise Fotovoltaik erfolgen. Ebenfalls sind gerichtete drahtlose Energieübertragungen anwendbar.

Neben den bereits erläuterten Vorteilen hinsichtlich der Einhaltung von Anforderungen an die Hygiene vermeldet die erfindungsgemäße Messeinrichtung spezielle Aufstelleinrichtungen und einen hierfür erforderlichen Stellplatz, Darüber hinaus ist es auch nicht erforderlich, die Messeinrichtung zu reinigen oder zu desinfizieren, da kein Körperkontakt mit dem Benutzer vorliegt.

Bei einer Kombination der Messeinrichtung mit einer Waage ist es Insbesondere auch möglich, bei bereits installierten Waagen den Installationsort des Emitters (14) und/ oder des Sensors (3) an die bereits gegebene Positionierung der Waage anzupassen.

## Patentansprüche

1. Vorrichtung zur Messung der Körperlänge einer Person, bei der im Bereich einer Innenbegrenzung eines Gebäuderaumes mindestens ein Emitter (14) zur Abgabe eines Primärsignales und mindestens ein Sensor (3) zur Erfassung eines Messsignals angeordnet sind und bei der sowohl der Emitter (14) als auch der Sensor (3) mit einer Steuereinrichtung gekoppelt sind, die mit einer Anzeigeeinrichtung gekoppelt ist, wobei der Sensor (3) im Bereich einer Decke (2) des Raumes (12) angeordnet und ein Projektor (17) zur Projektion einer Standmarkierung für die zu vermessende Person (5) verwendet ist, wobei sowohl der Emitter (14) als auch der Sensor (3) mit einer Steuereinrichtung (18) gekoppelt ist, die zusätzlich mit einer Anzeigeeinrichtung (19) gekoppelt ist, dass der Sensor (3) als eine Kamera ausgebildet und in einer vertikalen Richtung oberhalb der Person an der Decke (2) angeordnet ist **dadurch gekennzeichnet, dass** der Projektor (17) die Standmarkierung in einer vertikalen Richtung unterhalb der Kamera derart projiziert, dass sich eine vertikale Verbindungslinie von der Standmarkierung durch die Person hindurch bis in den Bereich der Kamera erstreckt, wobei sowohl der Sensor (3) als auch der Emitter (14) berührungslos mit der Steuereinrichtung (18) gekoppelt sind.

2. Vorrichtung nach Anspruch 1, **dadurch gekennzeichnet, dass** die Vorrichtung zusätzlich eine Waage zur Erfassung eines Gewichtes der zu vermessenden Person (5) aufweist.

3. Vorrichtung nach Anspruche 1 oder 2, **dadurch gekennzeichnet, dass** die Steuereinrichtung zur Auswertung einer Referenzmessung ausgebildet ist, die den Abstand des Sensors (3) zu einer Standfläche der zu vermessenden Person berücksichtigt.

4. Vorrichtung nach einem der Ansprüche 1 bis 3, **dadurch gekennzeichnet, dass** der Sensor (3) unter Verwendung einer Halterung installiert ist.

## Claims

1. Apparatus for measuring the height of a person, wherein at least one emitter (14) for emitting a primary signal and at least one sensor (3) for capturing a measurement signal are arranged in the region of an inner boundary of a room in a building and wherein both the emitter (14) and the sensor (3) are coupled to a control device coupled to a display device, wherein the sensor (3) is arranged in the region of a ceiling (2) of the room (12) and a projector (17) is used to project a standing mark for the person (5) to be measured, wherein both the emitter (14) and the sensor (3) are coupled to a control device (18) which is additionally coupled to a display device (19), in that the sensor (3) is embodied as a camera and arranged in a vertical direction above the person on the ceiling (2), **characterized in that** the projector (17) projects the standing mark in a vertical direction below the camera in such a way that a vertical connecting line extends from the standing mark through the person and to the region of the camera, wherein both the sensor (3) and the emitter (14) are contactlessly coupled to the control device (18).

2. Apparatus according to Claim 1, **characterized in that** the apparatus additionally comprises scales for capturing a weight of the person (5) to be measured.

3. Apparatus according to Claim 1 or 2, **characterized in that** control device is embodied to evaluate a reference measurement, which takes account of the distance of the sensor (3) from a standing area of the person to be measured.

4. Apparatus according to any one of Claims 1 to 3, **characterized in that** the sensor (3) is installed using a holder.

## Revendications

1. Dispositif servant à mesurer la longueur du corps d'une personne, dans lequel au moins un émetteur (14) pour délivrer un signal primaire et au moins un capteur (3) pour détecter un signal de mesure sont disposés dans la zone d'une limitation intérieure d'une pièce d'un bâtiment, et dans lequel aussi bien l'émetteur (14) que le capteur (3) sont couplés à un dispositif de commande qui est couplé à un dispositif d'affichage, dans lequel le capteur (3) est disposé au niveau d'un plafond (2) de la pièce (12) et un projecteur (17) est utilisé pour projeter un marquage de position debout pour la personne (5) à mesurer, dans lequel aussi bien l'émetteur (14) que le capteur (3) sont couplés à un dispositif de commande (18) qui est de plus couplé à un dispositif d'affichage (19), en ce que le capteur (3) est réalisé sous la forme d'une caméra et disposé au plafond (2) dans une direction verticale au-dessus de la personne,
**caractérisé en ce que** le projecteur (17) projette le marquage de position debout dans une direction verticale au-dessous de la caméra de telle sorte qu'une ligne de jonction verticale s'étend du marquage de position debout à travers la personne jusque dans la zone de la caméra, aussi bien le capteur (3) que l'émetteur (14) étant couplés sans contact au dispositif de commande (18).

2. Dispositif selon la revendication 1, **caractérisé en ce que** le dispositif présente en outre une balance pour détecter un poids de la personne (5) à mesurer.

3. Dispositif selon la revendication 1 ou 2, **caractérisé en ce que** le dispositif de commande est réalisé pour évaluer une mesure de référence qui tient compte de la distance du capteur (3) jusqu'à une surface de position debout de la personne à mesurer.

4. Dispositif selon l'une quelconque des revendications 1 à 3, **caractérisé en ce que** le capteur (3) est installé en utilisant un support.
